**Europäisches Patentamt**

**European Patent Office**

**Office européen des brevets**

(11) Veröffentlichungsnummer: **0 274 581 B1**

# EUROPÄISCHE PATENTSCHRIFT

(45) Veröffentlichungstag der Patentschrift: **19.02.92**

(51) Int. Cl.⁵: **A61K 9/50**

(21) Anmeldenummer: **87114857.3**

(22) Anmeldetag: **12.10.87**

Die Akte enthält technische Angaben, die nach dem Eingang der Anmeldung eingereicht wurden und die nicht in dieser Patentschrift enthalten sind.

(54) **Verfahren zur Herstellung von Liposomen.**

(30) Priorität: **18.10.86 DE 3635506**

(43) Veröffentlichungstag der Anmeldung:
**20.07.88 Patentblatt 88/29**

(45) Bekanntmachung des Hinweises auf die Patenterteilung:
**19.02.92 Patentblatt 92/08**

(84) Benannte Vertragsstaaten:
**AT BE CH FR GB IT LI NL SE**

(56) Entgegenhaltungen:
**WO-A-85/01440**
**WO-A-86/01102**

(73) Patentinhaber: **MPI Medizinische und pharmazeutische Innovationen GmbH**
**Bopserwaldstrasse 26**
**W-7000 Stuttgart 1(DE)**

(72) Erfinder: **Die Erfinder haben auf ihre Nennung verzichtet**

(74) Vertreter: **Patentanwälte Phys. Bartels**
**Dipl.-Ing. Fink Dr.-Ing. Held**
**Lange Strasse 51**
**W-7000 Stuttgart 1(DE)**

**Beschreibung**

Die Erfindung betrifft ein Verfahren zur Herstellung liposomal eingeschlossener Wirkstoffe unter Verwendung von aus amphiphilen Substanzen hergestellten Liposomen.

Liposomen sind künstliche hohlkugelförmige Partikel, auch Vesikel genannt. Die Membran solcher Vesikel, bei denen es sich um multilamellare große Vesikel, kleine unilamellare Vesikel und große unilamellare Vesikel handeln kann, besteht aus einer oder mehreren Lamellen geeigneter Lipide. Meistens handelt es sich hierbei um Phospholipide.

Bei den bekannten Verfahren der eingangs genannten Art wird die Präparation der Liposomen und der Einschluß der Wirkstoffe gleichzeitig durchgeführt. Dies hat zur Folge, daß bei höheren Einschlußraten oft gravierende Nachteile in Kauf genommen werden müssen. Zum einen kann die Anwesenheit der einzuschließenden Wirkstoffe die Bildung der Liposomen stören, so daß Produkte mit unerwünschten Eigenschaften entstehen. Insbesondere kann dabei die Lamellarität der entstehenden Liposomen oder die Größe beeinflußt werden. Die Kontrolle der Präparation und die Eigenschaft der Liposomen sind aber eine unerläßliche Voraussetzung für die Anwendungssicherheit von liposomal eingeschlossenen Wirkstoffen. Zum anderen können durch die verschiedenen Verfahren zur Herstellung der Liposomen die einzuschließenden Wirkstoffe selbst ungünstig verändert verden. Als besonders nachteilig für empfindliche Wirkstoffe haben sich mechanische Einwirkungen, zum Beispiel hohe Drucke oder Ultraschall, herausgestellt. Ferner können organische Lösungsmittel oder hohe Detergenskonzentrationen Denaturierungen der einzuschließenden Wirkstoffe verursachen. Für eine erfolgreiche Anwendung liposomal eingeschlossener Wirkstoffe ist aber sowohl ein intakter Wirkstoff als auch ein intaktes, nicht toxisches liposomales Carriersystem unerläßliche Vorraussetzung. Hinzu kommt noch, daß die meisten der bekannten Verfahren eine niedrige Einschlußeffektivität aufweisen. Wo die Ausbeute hoch ist, ist sie meist nur für bestimmte Substanzklassen zu erzielen, eine allgemeine Anwendbarkeit also nicht gegeben. Schließlich besteht bei manchen bekannten Verfahren die Gefahr, daß die erforderlichen unphysiologischen organischen Lösungsmittel zum Teil in der Liposomenmembran verbleiben und damit die Endprodukte toxische Wirkung auf den Organismus oder Einzelzellen entfalten können.

Es ist auch bekannt (WO-A 86/01 102), aktive Substanzen unter Ausnutzung eines Membranpotentials in vorgefertigte Liposomen einzuschleusen. Dieses Verfahren kann jedoch aufgrund der technischen Gegebenheiten nur für lipophile Substanzen, die in geladenem Zustand vorliegen, angewendet werden.

Weiterhin ist ein Verfahren bekannt (WO-A-85 01 440), das sich speziell zur Aufnahme macromolekularer Substanzen in Vesikel eignet. Dabei werden Liposomen in einem ersten Schritt in Gegenwart der einzuschließenden Substanz hydratisiert und anschließend durch Zugabe eines Detergens die Struktur der Liposomen verändert.

Der Erfindung liegt die Aufgabe zugrunde, ein Verfahren zur Herstellung liposomal eingeschlossener Wirkstoffe zu schaffen, das frei von den genannten Nachteilen der bekannten Verfahren ist und insbesondere eine Änderung der Liposomenstruktur infolge der Wirkstoffzugabe vermeidet. Diese Aufgabe löst ein Verfahren mit den Merkmalen des Anspruches 1.

Durch die Verwendung vorgefertigter Liposomen, die aus nicht toxischen, natürlichen oder künstlichen Membranlipiden hergestellt werden können, lassen sich die Präparation und die Eigenschaften der Liposomen ungestört kontrollieren. Ferner kann während des Einschlußvorgangs der Wirkstoffe die membrangebundene Detergenskonzentration so niedrig gehalten werden, daß eine Emulgierung der Liposomenlipide oder sonstige Veränderungen an der Liposomenstruktur vermieden oder unerheblich klein gehalten werden kann. Die Forderung nach einem intakten, nicht toxischen liposomalen Carriersystem läßt sich deshalb in vollem Umfang erfüllen. Weitere Vorteile des erfindungsgemäßen Verfahrens bestehen darin, daß es billig ist und mit geringem zeitlichen Aufwand durchgeführt werden kann. Wesentlich ist ferner, daß die vorgefertigten Liposomen mit Einschlußraten bis über 30% gefüllt werden können, wobei als einzuschließende Wirkstoffe eine Vielzahl von chemisch und physikalisch unterschiedlichen Substanzen, insbesondere alle hydrophilen chemischen Substanzen bis zu einem Molekulargewicht von 80 000 Dalton in Frage kommt. Bedeutsam ist schließlich auch noch, daß die Wirkstoffe bei der Beschickung nicht irreversibel verändert werden und daß das liposomale Wirkstoffsystem keine unerwünschten Substanzen erhält. Das erfindungsgemäße Verfahren erlaubt daher die Bereitstellung nicht toxischer liposomaler Wirkstoffe für die Anwendung als Arzneimittel mit maßgeschneiderten Eigenschaften, hoher Einschlußrate und ohne Beeinträchtigung des Wirkstoffes nach einem allgemeinen Prinzip. Das erfindungsgemäße Verfahren erweitert deshalb die Anwendbarkeit liposomal eingeschlossener Wirkstoffe im pharmazeutischen und naturwissenschaftlichen Bereich.

Vorzugsweise wird die MembranPermeabitität nach der Verteilung der Wirkstoffes oder der Wirkstoffe auf das Außen- und Innenvolumen der Liposomen rasch gesenkt. Der in die Liposomen eingeschleuste Wirkstoff bleibt dann im Liposom eingeschlossen.

Besonders vorteilhaft sind Detergentien amphiphilen Charakters, da sie nach dem Wirkstoffeinschluß nahezu vollständig mit wässrigen Medien entfernt werden können, zum Beispiel durch Dialyse oder Gelfiltration. Bei Verwendung geeigneter Detergentien, zum Beispiel Gallensäuren, sind verbleibende Spuren von diesen in den Liposomen ungiftig.

Die Einschlußeffektivität hängt bei dem erfindungsgemäßen Verfahren im wesentlich vom Anteil des Innenvolumens der Liposomen am Gesamtverteilungsvolumen des Wirkstoffes ab. Sehr hohe Einschlußraten können durch Konzentrierung der Liposomen erreicht werden. Im Zustand hoher Lipidkonzentration verteilt sich ein zugegebenes Detergens hauptsächlich in die Lipidmembran. Die Detergenskonzentration im wässrigen Volumenanteil bleibt dabei so gering, daß eine Schädigung der einzuschließenden Substanzen unwahrscheinlich ist.

Die Liposomen können in bekannter Weise nach sieben grundsätzlich verschiedenen Verfahren hergestellt werden, nämlich durch mechanische Einwirkung auf wässrige Lipidsuspensionen, durch temperaturabhängige Veränderungen wässriger Lipidsuspensionen, durch Veränderung der Protonenkonzentration, durch Wechselwirkung mit Elektroden, durch Verwendung organischer Lösungsmittel, durch die Einwirkung von Detergentien auf Lipide in wässrigen Systemen sowie durch Fusion fertiger Liposomen. Auch eine Kombination verschiedener Verfahren ist möglich.

Im folgenden ist die Erfindung anhand verschiedener Ausführungsbeispiele weiter erläutert.

Beispiel 1

Aus Eigelb-Lezithin (Sigma) und n-Oktyl-$\beta$-D-Glukopyranosid (Sigma) wurden in Phosphatpuffer (10mM Phosphat, 150mM NaCl, pH 7,4) mittels Detergensdialyse (Literatur Milsmann) homogene unilamellare große Liposomen hergestellt. Die Liposomen hatten einen mittleren Durchmesser von 180 nm. Die Liposomen wurden durch Ultrazentrifugation pellettiert und mit Puffer auf 55 mg Lipid/ml resuspendiert.
Zu 100 $\mu$l Liposomensuspension wurden 10 $\mu$l 200 mmolare Natriumcholat-Lösung in Puffer und 10 $\mu$l wässrige H-Inulinlösung (NEN, Molekulargewicht ca. 5000 Daltons, 1,5 $\mu$Ci) zugefügt. Die Suspension wurde sofort gründlich gemischt und bei Zimmertemperatur 15 min inkubiert. Nach Verdünnung mit 120 $\mu$l Puffer wurde säulenchromatographisch über Sepharose 2B-CL (Pharmacia) das nicht eingeschlossene Inulin und das Cholat von liposomal eingeschlossenem Inulin abgetrennt.
Die Verteilung der $^3$H-Aktivität ergab einen Anteil des liposomalen Inulin am gesamten eingesetzten Inulin von 29,2 %. In einem Parallelversuch wurde[3]H-markierte Cholatlösung verwendet und statt Inulinlösung nur Wasser. Nach Pellettierung der Liposomen durch Ultrazentrifugation wurde im Überstand der Anteil an nicht liposomengebundenem Cholat bestimmt. Er betrug 23 %, d.h. von der Cholat-Gesamtkonzentration von 18,2 mM betrug die freie Cholatkonzentration lediglich 4,2 mM.

Beispiel 2

Statt der hochverdünnten[3]H-Inulinlösung in Beispiel 1 wurden 10 $\mu$l einer konzenrierten wässrigen Inulinlösung zugegeben (20 mg/ml mit 1,5 $\mu$Ci[3]H). Die Einschlußeffektivität betrug 31,7 %.

Beispiel 3

Statt der[3]H-Inulinlösung in Beispiel 1 wurde $^3$H-Dextranlösung zugegeben (1,5 $\mu$Ci, mittl. Molekulargewicht 70000 Daltons, Amersham). Die Einschlußeffektivität betrug 31,1 %.

Beispiel 4

Aus Eigelb-Lezithin und Natriumcholat wurden wie in Beispiel 1 homogene unilamellare Liposomen hergestellt mit einem mittleren Durchmesser von 71 nm. Die Liposomen wurden durch Ultrazentrifugation pellettiert und mit Puffer auf 210 mg Lipid/ml resuspendiert.
Zu 100 $\mu$l Liposomensuspension wurden wie in Beispiel 1 Cholat-und Inulinlösung zugegeben. Die Einschlußeffektivität betrug 17,1 %.

Beispiel 5

Aus 30 Mol% Eigelb-Lezithin und 70 Mol% Phosphatidylserin aus Rinderhirn (Sigma) und n-Oktyl-$\beta$-D-Glucopyranosid wurden wie in Beispiel 1 homogene unilamellare Liposomen hergestellt mit einem mittleren Durchmesser von 77 nm. Der Einschluß von $^3$H-Inulin wurde wie in Beispiel 1 durchgeführt, jedoch mit dem doppelten Volumen an Cholatlösung. Die Einschlußeffektivität betrug 8,3 %.

Beispiel 6

Statt $^3$H-Inulin wie in Beispiel 5 wurde eine Lösung von ATP (Sigma) zugegeben(2 mg in 10 $\mu$l, 0,2 $\mu$Ci[3]H-ATP, NEN). Die Einschlußeffektivität betrug 8,3 %.

Beispiel 7

Statt $^3$H-Inulin wie in Beispiel 5 wurde eine wässrige Polyuridin-Lösung zugegeben (Sigma, Molekulargewicht 10000, 1 mg / 10 $\mu$l, 2 $\mu$Ci $^3$H-Poly-U, NEN). Die Einschlußeffektivität betrug 3,5 %.

**Patentansprüche**

1. Verfahren zur Herstellung liposomal eingeschlossener Wirkstoffe unter Verwendung von aus amphiphilen Substanzen hergestellten Liposomen, dadurch gekennzeichnet, daß an vorgefertigten Liposomen durch Zugabe wenigstens eines für den mit dem Wirkstoff oder den Wirkstoffen zu behandelnden Organismus unbedenklichen Detergens, wie z. B. Gallensäuren oder Natriumcholat, in einer Konzentration, bei der eine Emulgierung der Liposomenlipide oder sonstige Veränderungen an der Liposomenstruktur vermieden oder unerheblich klein gehalten werden, eine vorübergehende Erhöhung der Membranpermeabilität bewirkt und während dieser Phase erhöhter Membranpermeabilität der Eintritt des Wirkstoffes oder der Wirkstoffe aus einem die Liposomen enthaltenden Außenmedium in den Innenraum der Liposomen herbeigeführt wird.

2. Verfahren nach Anspruch 1, dadurch gekennzeichnet, daß die Membronpermeabilität nach der Verteilung des Wirkstoffes oder der Wirkstoffe auf das Außen- und Innenvolumen der Liposomen rasch gesenkt wird.

3. Verfahren nach Anspruch 1 oder 2, dadurch gekennzeichnet, daß Detergentien amphiphilen Charakters verwendet werden.

4. Verfahren nach Anspruch 3, dadurch gekennzeichnet, daß das Detergens oder die Detergentien zumindest nahezu vollständig mit wässrigen Medien entfernt werden.

5. Verfahren nach Anspruch 4, dadurch gekennzeichnet, daß die Entfernung der Detergentien durch Dialyse, Filtration, Gelfiltration, Verdünnung oder Auswaschen erfolgt.

6. Verfahren nach einem der Ansprüche 1 bis 5, dadurch gekennzeichnet, daß der Anteil des Innenvolumens der Liposomen am gesamten Verteilungsvolumen des Wirkstoffes in Abhän-gigkeit von der angestrebten Einschlußeffektivi-tät gewählt wird.

7. Verfahren nach einem der Ansprüche 1 bis 6, dadurch gekennzeichnet, daß die Konzentrierung der Liposomen in Abhängigkeit von der angestrebten Einschlußrate gewählt wird.

8. Verfahren nach einem der Ansprüche 1 bis 7, dadurch gekennzeichnet, daß uni- oder oligolamellare Liposomen verwendet werden.

9. Verfahren nach einem der Ansprüche 1 bis 8, dadurch gekennzeichnet, daß eine Auswahl aus Liposomen verschiedener Lipidkompositionen getroffen wird.

**Claims**

1. Process for preparing active substances enclosed within liposomes using liposomes pro-duced from amphophylic substances, charac-terized in that the membrane permeability is temporarily increased on pre-prepared liposomes by the addition of at least one detergent, such as bile acid or sodium cholate, which is not harmful to the organism to be treated with the active substance or substances, in a concentration such that emulsification of the liposome lipids or other changes to the liposome structure are avoided or kept very small, and during this phase of increased membrane permeability the introduction of the active substance or substances from an external medium containing the liposomes into the interior of the liposomes is brought about.

2. Process according to Claim 1, characterized in that the membrane permeability is rapidly lowered after the active substance or substances has/have been distributed on the outer and inner volumes of the liposomes.

3. Process according to Claim 1 or 2, characterized in that detergents of an amphophylic nature are used.

4. Process according to Claim 3, characterized in that the detergent or detergents is/are removed at least substantially completely with aqueous media.

5. Process according to Claim 4, characterized in that the detergents are removed by means of dialysis, filtration, gel filtration, dilution or washing.

6. Process according to any one of Claims 1 to 5, characterized in that the proportion of the inner volume of the liposomes relative to the entire distribution volume of the active substance is selected in dependence upon the desired degree of efficiency of the enclosure.

7. Process according to any one of Claims 1 to 6, characterized in that the concentration of the liposomes is selected in dependence upon the desired degree of enclosure.

8. Process according to any one of Claims 1 to 7, characterized in that uni- or oligolamellar liposomes are used.

9. Process according to any one of Claims 1 to 8, characterized in that liposomes of different lipid compositions are selected.

**Revendications**

1. Procédé pour inclure des substances actives dans des liposomes par utilisation de liposomes préparés à partir de substances amphiphiles, caractérisé en ce que, sur des liposomes formés au préalable, on provoque, par addition d'au moins un détergent non toxique pour l'organisme à traiter par la ou les substances actives, par exemple des acides biliaires ou du cholate de sodium, à une concentration évitant ou maintenant dans une mesure négligeable une mise en émulsion des lipides des liposomes ou d'autres altérations de la structure des liposomes, une augmentation transitoire de la perméabilité des membranes et, pendant cette phase de perméabilité accrue des membranes, on fait entrer dans le volume intérieur des liposomes la ou les substances actives provenant d'un milieu extérieur contenant les liposomes.

2. Procédé selon la revendication 1, caractérisé en ce que, après la répartition de la ou des substances actives dans le volume extérieur et intérieur des liposomes, on diminue rapidement la perméabilité des membranes.

3. Procédé selon la revendication 1 ou 2, caractérisé en ce que l'on utilise des détergents à caractère amphiphile.

4. Procédé selon la revendication 3, caractérisé en ce que le ou les détergents sont éliminés en quasi-totalité au moyen de milieux aqueux.

5. Procédé selon la revendication 4, caractérisé en ce que les détergents sont éliminés par dialyse, filtration, filtration sur gel, dilution ou extraction par lavage.

6. Procédé selon l'une des revendications 1 à 5, caractérisé en ce que l'on choisit le rapport du volume intérieur des liposomes au volume de répartition total de la substance active en fonction de l'efficacité d'inclusion voulue.

7. Procédé selon une des revendications 1 à 6, caractérisé en ce que l'on choisit la concentration des liposomes en fonction des taux d'inclusion voulus.

8. Procédé selon une des revendications 1 à 7, caractérisé en ce que l'on utilise des liposomes uni- ou oligo-lamellaires.

9. Procédé selon une des revendications 1 à 8, caractérisé en ce que l'on choisit des liposomes à compositions variées en lipides.